# EUROPEAN PATENT APPLICATION

(11) **EP 1 661 990 A1**
(43) Date of publication of application: **31.05.2006**
(21) Application number: 04748303.7
(22) Date of filing: 06.08.2004
(51) Int. Cl.: C12N 15/09, C07K 14/82, C12P 21/08, C07K 16/32, C12N 15/12, C12N 1/15, C12N 1/19, C12N 1/21, C12N 5/10, C12P 21/02, C12Q 1/68, A61K 31/7088, A61K 48/00, A61P 35/00, G01N 33/574

(54) **POLYPEPTIDE SPECIFIC TO LIVER CANCER, POLYNUCLEOTIDE ENCODING THE POLYPEPTIDE AND RNA MOLECULE INHIBITING THE EXPRESSION OF THE POLYPEPTIDE**

(30) Priority: 11.08.2003 JP 2003291060
(71) Applicant: Kureha Corporation, Tokyo 103-8552 (JP); Mukaida, Naofumi, Kanazawa-shi, Ishikawa 9218105 (JP); Hujii, Chifumi, Kanazawa-shi, Ishikawa 9201167 (JP)
(72) Inventor: MUKAIDA, Naofumi, Kanazawa-shi, Ishikawa 9218105 (JP); HUJII, Chifumi, Kanazawa-shi, Ishikawa 9201167 (JP); HIROSE, Kunitaka, Nerima-ku, Tokyo 1790074 (JP)
(74) Representative: Jones, Helen M.M.
(86) International application number: PCT/JP2004/011669
(87) International publication number: WO 2005/014809

(57) **Abstract**

There are provided a polypeptide specific to liver cancer, a polynucleotide coding for the polypeptide, and an RNA molecule suppressing the expression of the polypeptide. The present invention is particularly intended for a polypeptide comprising the amino acid sequence of SEQ ID NO: 1; a polypeptide having at least 80% homology to the amino acid sequence of SEQ ID NO: 1 and having immunogenicity inducing the production of an antibody against the polypeptide comprising the amino acid sequence of SEQ ID NO: 1; nucleotide fragments thereof; polynucleotides coding for the polypeptides and fragments thereof; and an RNA molecule having a partial sequence corresponding to mRNA coding for the polypeptide comprising the amino acid sequence of SEQ ID NO: 1.

SEQUENCE LISTING

<110>  Kureha Chemical Industry Company Limited

<120>  Polypeptide associated with hepatocellular carcinoma, polynucleotide
encoding the polypeptide and RNA molecule supressing the polypeptide expression

<130>  HMJ04175EP

<140>  EP 04748303.7
<141>  2004-08-06

<160>  11

<170>  PatentIn version 3.1

<210>  1
<211>  326
<212>  PRT
<213>  Homo sapiens

<400>  1

Met Leu Leu Ser Lys Phe Gly Ser Leu Ala His Leu Cys Gly Pro Gly
1               5                   10                  15

Gly Val Asp His Leu Pro Val Lys Ile Leu Gln Pro Ala Lys Ala Asp
            20                  25                  30

Lys Glu Ser Phe Glu Lys Ala Tyr Gln Val Gly Ala Val Leu Gly Ser
        35                  40                  45

Gly Gly Phe Gly Thr Val Tyr Ala Gly Ser Arg Ile Ala Asp Gly Leu
    50                  55                  60

Pro Val Ala Val Lys His Val Val Lys Glu Arg Val Thr Glu Trp Gly
65                  70                  75                  80

Ser Leu Gly Gly Ala Thr Val Pro Leu Glu Val Val Leu Leu Arg Lys
                85                  90                  95

Val Gly Ala Ala Gly Gly Ala Arg Gly Val Ile Arg Leu Leu Asp Trp
            100                 105                 110

Phe Glu Arg Pro Asp Gly Phe Leu Leu Val Leu Glu Arg Pro Glu Pro
        115                 120                 125

Ala Gln Asp Leu Phe Asp Phe Ile Thr Glu Arg Gly Ala Leu Asp Glu
    130                 135                 140

Pro Leu Ala Arg Arg Phe Phe Ala Gln Val Leu Ala Ala Val Arg His
145                 150                 155                 160

Cys His Ser Cys Gly Val Val His Arg Asp Ile Lys Asp Glu Asn Leu
                165                 170                 175

Leu Val Asp Leu Arg Ser Gly Glu Leu Lys Leu Ile Asp Phe Gly Ser
            180                 185                 190

Gly Ala Leu Leu Lys Asp Thr Val Tyr Thr Asp Phe Asp Gly Thr Arg
        195                 200                 205

Val Tyr Ser Pro Pro Glu Trp Ile Arg Tyr His Arg Tyr His Gly Arg
    210                 215                 220

Ser Ala Thr Val Trp Ser Leu Gly Val Leu Leu Tyr Asp Met Val Cys
225                 230                 235                 240

Gly Asp Ile Pro Phe Glu Gln Asp Glu Glu Ile Leu Arg Gly Arg Leu
                245                 250                 255

Leu Phe Arg Arg Arg Val Ser Pro Glu Cys Gln Gln Leu Ile Arg Trp
            260                 265                 270

Cys Leu Ser Leu Arg Pro Ser Glu Arg Pro Ser Leu Asp Gln Ile Ala
        275                 280                 285

Ala His Pro Trp Met Leu Gly Ala Asp Gly Gly Ala Pro Glu Ser Cys
    290                 295                 300

Asp Leu Arg Leu Cys Thr Leu Asp Pro Asp Asp Val Ala Ser Thr Thr
305                 310                 315                 320

Ser Ser Ser Glu Ser Leu
                325

<210>  2
<211>  2392
<212>  DNA
<213>  Homo sapiens

<400>  2
agcggaccga cgcgacacgc cgtgcgcctc cgcggctgcg ctacgaaaac gagtcccgga      60

gcggccccgc gcccgccgca cccggccctc gcccacccga agacaggcgc ccagctgccc     120

cgccgtctcc ccagctagcg cccggccgcc gccgcctcgc gggccccggg cggaaggggg     180

cggggtcccg attcgccccg cccccgcgga gggatacgcg gcgccgcggc ccaaaacccc     240

cgggcgaggc ggccggggcg ggtgaggcgc tccgcctgct gctcgtctac gcggtccccg     300

cgggccttcc gggcccactg cgccgcgcgg accgcctcgg gctcggacgg ccggtgtccc 360
cggcgcgccg ctcgcccgga tcggccgcgg cttcggcgcc tggggctcgg ggctccgggg 420
aggccgtcgc ccgcgatgct gctctccaag ttcggctccc tggcgcacct ctgcgggccc 480
ggcggcgtgg accacctccc ggtgaagatc ctgcagccag ccaaggcgga caaggagagc 540
ttcgagaagg cgtaccaggt gggcgccgtg ctgggtagcg gcggcttcgg cacggtctac 600
gcgggtagcc gcatcgccga cgggctcccg gtggctgtga agcacgtggt gaaggagcgg 660
gtgaccgagt ggggcagcct gggcggcgcg accgtgcccc tggaggtggt gctgctgcgc 720
aaggtgggcg cggcgggcgg cgcgcgcggc gtcatccgcc tgctggactg gttcgagcgg 780
cccgacggct tcctgctggt gctggagcgg cccgagccgg cgcaggacct cttcgacttt 840
atcacggagc gcggcgccct ggacgagccg ctggcgcgcc gcttcttcgc gcaggtgctg 900
gccgccgtgc gccactgcca cagctgcggg gtcgtgcacc gcgacattaa ggacgaaaat 960
ctgcttgtgg acctgcgctc cggagagctc aagctcatcg acttcggttc gggtgcgctg 1020
ctcaaggaca cggtctacac cgacttcgac ggcacccgag tgtacagccc cccggagtgg 1080
atccgctacc accgctacca cggcgctcgg gccaccgtgt ggtcgctggg cgtgcttctc 1140
tacgatatgg tgtgtgggga catccccttc gagcaggacg aggagatcct ccgaggccgc 1200
ctgctcttcc ggaggagggt ctctccagag tgccagcagc tgatccggtg gtgcctgtcc 1260
ctgcggccct cagagcggcc gtcgctggat cagattgcgg cccatccctg gatgctgggg 1320
gctgacgggg gcgccccgga gagctgtgac ctgcggctgt gcaccctcga ccctgatgac 1380
gtggccagca ccacgtccag cagcgagagc ttgtgaggag ctgcacctga ctgggagcta 1440
ggggaccacc tgccttggcc agacctggga cgcccccaga ccctgacttt ttcctgcgtg 1500
ggccgtctcc tcctgcggaa gcagtgacct ctgacccctg gtgaccttcg ctttgagtgc 1560
cttttgaacg ctggtcccgc gggacttggt tttctcaagc tctgtctgtc caaagacgct 1620
ccggtcgagg tcccgcctgc cctgggtgga tacttgaacc ccagacgccc ctctgtgctg 1680
ctgtgtccgg aggcggcctt cccatctgcc tgcccacccg gagctctttc cgccggcgca 1740
gggtcccaag cccacctccc gccctcagtc ctgcggtgtg cgtctgggca cgtcctgcac 1800
acacaatgca agtcctggcc tccgcgcccg cccgcccacg cgagccgtac ccgccgccaa 1860
ctctgttatt tatggtgtga ccccctggag gtgccctcgg cccaccgggg ctatttattg 1920
tttaatttat ttgttgaggt tatttcctct gagcagtctg cctctcccaa gccccagggg 1980
acagtgggga ggcaggggag ggggtggctg tggtccaggg accccaggcc ctgattcctg 2040
tgcctggcgt ctgtcctggc cccgcctgtc agaagatgaa catgtatagt ggctaactta 2100

aggggagtgg gtgaccctga cacttccagg cactgtgccc agggtttggg ttttaaatta 2160

ttgactttgt acagtctgct tgtgggctct gaaagctggg gtggggccag agcctgagcg 2220

tttaatttat tcagtacctg tgtttgtgtg aatgcggtgt gtgcaggcat cgcagatggg 2280

ggttctttca gttcaaaagt gagatgtctg gagatcatat ttttttatac aggtatttca 2340

attaaaatgt ttttgtacat aaaaaaaaaa aaaaaaaaaa aaaaaaaaaa aa 2392

<210> 3
<211> 28
<212> DNA
<213> Artificial sequence

<220>
<223> Pim-3 sense primer

<400> 3
aagcagtgac ctctgacccc tggtgacc 28

<210> 4
<211> 25
<212> DNA
<213> Artificial sequence

<220>
<223> Pim-3 antisense primer

<400> 4
cagcggaacc gctcattgcc aatgg 25

<210> 5
<211> 20
<212> DNA
<213> Artificial sequence

<220>
<223> GAPDH sense prime

<400> 5
accacagtcc atgccatcac 20

<210> 6
<211> 20
<212> DNA
<213> Artificial sequence

<220>
<223> GAPDH antisense primer

<400> 6
tccaccaccc tgttgctgta 20

<210> 7
<211> 20

<212>  DNA
<213>  Artificial sequence

<220>
<223>  Probe used in first screening of hPim-3

<400>  7
ctgtgaagca cgtggtgaag                                                    20


<210>  8
<211>  19
<212>  PRT
<213>  Artificial sequence

<220>
<223>  Epitop used in the production of antibody to hPim-3 polypeptide

<400>  8

Cys Gly Pro Gly Gly Val Asp His Leu Pro Val Lys Ile Leu Gln Pro
1               5                   10                  15


Ala Lys Ala



<210>  9
<211>  21
<212>  DNA
<213>  Artificial sequence

<220>
<223>  Targeted mRNA

<400>  9
gcacgtggtg aaggagcgcg g                                                  21


<210>  10
<211>  21
<212>  RNA
<213>  Artificial sequence

<220>
<223>  siRNA in example 9

<400>  10
ccgcgcuccu ucaccacgug c                                                  21


<210>  11
<211>  19
<212>  RNA
<213>  Artificial sequence

<220>
<223>  Random RNA fragment

<400>  11

gcgcgcuuug uaggauucg 19

## Description

### Technical Field

The present invention relates to a novel polypeptide specific to liver cancer, a polynucleotide coding for the polypeptide, and an RNA molecule suppressing the expression of the polypeptide. In particular, the present invention relates to a novel polypeptide specific to liver cancer coded for a human proto-oncogene Pim-3; an antibody specific thereto; a diagnostic kit; a polynucleotide coding for the specific polypeptide; a vector comprising the polynucleotide or a fragment thereof; a transfectant; and an RNA molecule capable of specifically suppressing the expression of the specific polypeptide and being applied to the treatment of liver cancer.

### Background Art

Liver cancer is cancer commonly found in Southeast Asia, Africa, and Southern Europe, which accounts for the 8th leading cause of death attributed to cancers, while its incidence is also high in Japan, especially in western Japan. The primary cause of this liver cancer is considered to be chronic hepatitis and cirrhosis caused by persistent infection with hepatitis B or C viruses (Non-Patent Document 1).

For example, blood tests utilizing tumor markers such as α-fetoprotein (AFP) and PIVKA-II as well as diagnostic imaging such as X-ray examinations, ultrasonography, CT, MRI, and angiography are performed as diagnostic methods for this liver cancer.
However, because the tumor markers such as α-fetoprotein (AFP) exhibit a relatively high value in the presence of chronic hepatitis or cirrhosis, even in the absence of liver cancer, the tests utilizing such a tumor marker require reexamination or must be used in combination with diagnostic imaging and followed up. In addition, there are cases where primary liver cancer, if any, is not detected in the tests. Alternatively, because the diagnostic imaging provides hard-to-view or blind spots for some persons, it presents such a problem as an oversight unless several examinations are combined. Therefore, there is a need for a method to examine liver cancer with higher accuracy and efficiency.

For example, the resection of liver cancer by surgery, microwave coagulation therapy (MCT), radiofrequency ablation (RFA), percutaneous ethanol injection therapy (PEIT), hepatic artery embolization, chemotherapy by the hepatic intraarterial injection of anticancer drugs with reservoirs, and radiation are performed as therapeutic methods for liver cancer. However, many patients with liver cancer have a deteriorated liver function resulting from their liver cancer complicated with chronic liver disease such as chronic hepatitis or cirrhosis, and are subject to constraints on therapeutic methods. Consequently, in not a few cases, they can not receive surgery and besides, can merely choose minimally invasive therapeutic methods with a small load on the liver. Therefore, there is a need for therapeutic methods with a small load that can be applied even to patients having a deteriorated liver function.

With recent significant advances in molecular biology and genetic engineering, novel tumor markers, antibodies, or small-interfering RNAs (siRNA) have been reported for a variety of cancers (Patent Documents 1 and 2). Accordingly, it is desired to develop excellent diagnostic and therapeutic methods for liver cancer on the basis of similar studies.

[Patent Document 1] International Publication pamphlet of WO 99/032619
[Patent Document 2] International Publication pamphlet of WO 01/075164
[Non-Patent Document 1] Geller, S. A. 2002. Hepatitis B and hepatitis C. Clin. Liver Dis. 6: 317-334.

### Disclosure of the Invention

An object of the present invention is to provide a novel polypeptide specific to liver cancer, a polynucleotide coding for the polypeptide, and an RNA molecule suppressing the expression of the polypeptide, which lead to the development of a novel diagnostic method, diagnostic drug, and therapeutic drug for liver cancer.

Hepatitis virus infection considered to be a cause of liver cancer develops cytolytic T lymphocytes (CTL) specific to viral antigens, which influence both virus eradication and liver failure. In addition, a cycle of liver cell destruction and regeneration in which CTL is involved is considered to create a mitogenic and mutagenic environment that leads to liver cancer. This hypothesis is indicated by the report by Nakamoto et al., in which HBV antigen (HBs) transgenic mice (HBsTg) with the bone marrow removed received the transplantation of bone marrow cells and spleen cells from syngeneic wild-type mice immunized with HBs and, 15 months after the transplantation, developed liver cancer (*1). However, a molecular mechanism basically responsible for this liver cancer development has not fully been examined yet.

Heretofore, evidence suggesting that the initial development and malignant alteration of tumors result from accumulated changes in the expression and structures of various genes has been reported one after another. In the liver cancer model of HBsTg, the long-term latency of the symptom also suggests the presence of complex changes similar to the changes. Transcriptome analysis is conducted on various types of tumors including liver cancer in order to elucidate widespread genomic changes in gene expression. Most of liver cancer researches have compared a gene expression pattern between a tumor site and a non-tumor site in the liver derived from the same patient. However, because the complex changes are expected in this non-tumor site as compared with normal tissue, this type of analysis had the possibility that some changes in gene expression, which are common to the tumor site and the non-tumor site that is not normal tissue, can not be detected. Fifteen months after the transplantation, a non-tumor site where the dysplasia of liver cells could be confirmed and a tumor site coexisted in the liver of HBsTg. Therefore, this non-tumor site was used as a lesion in a precancerous condition, and gene expression patterns were compared using fluorescent differential display (FDD) between the non-tumor site and normal tissue.

Namely, the present inventors identified some genes that were expressed in the lesion in a precancerous condition in the liver cancer model of HBsTg, and studied, among these genes, a proto-oncogene whose expression in the liver and human homologue were heretofore unreported. As a result, the present inventors gained findings that a Pim-3 gene codes for a protein specific to human liver cancer, and consequently completed the present invention by determining an amino acid sequence of its polypeptide and a polynucleotide sequence of the DNA and studying their properties.

Thus, the present invention provides a polypeptide comprising the amino acid sequence of SEQ ID NO: 1 as well as a polypeptide having at least 80% homology to the amino acid sequence of SEQ ID NO: 1; having an amino acid sequence, wherein at least an amino acid residue at position 39, 84, 296, or 300 from the N-terminus is Ala, an amino acid residue at position 85 is Thr, an amino acid residue at position 163 or 333 is Ser, an amino acid residue at position 195 or 257 is Leu, an amino acid residue at position 271 is Arg, an amino acid residue at position 297 is Asp, an amino acid residue at position 313 is Pro, or an amino acid residue at position 316 is Val, in correspondence with the amino acid sequence of SEQ ID NO: 1; and having immunogenicity inducing the production of an antibody against the polypeptide comprising the amino acid sequence of SEQ ID NO: 1. The polypeptides include those isolated.

In addition, the present invention provides a polypeptide fragment having a partial sequence of the amino acid sequence of SEQ ID NO: 1; or a partial sequence of an amino acid sequence having at least 80% homology to the amino acid sequence of SEQ ID NO: 1, wherein at least an amino acid residue at position 39, 84, 296, or 300 from the N-terminus is Ala, an amino acid residue at position 85 is Thr, an amino acid residue at position 163 or 333 is Ser, an amino acid residue at position 195 or 257 is Leu, an amino acid residue at position 271 is Arg, an amino acid residue at position 297 is Asp, an amino acid residue at position 313 is Pro, or an amino acid residue at position 316 is Val, in correspondence with the amino acid sequence of SEQ ID NO: 1; and having immunogenicity inducing the production of an antibody against the polypeptide comprising the amino acid sequence of SEQ ID NO: 1. The polypeptide fragment includes those isolated.

Moreover, the present invention provides a composition for producing an antibody specific to the polypeptide comprising the amino acid sequence of SEQ ID NO: 1, comprising at least one selected from the group consisting of the polypeptides and the polypeptide fragment.

Moreover, the present invention provides a method for the production of the antibody specific to the polypeptide comprising the amino acid sequence of SEQ ID NO: 1, comprising administering the composition to a mammal.

Moreover, the present invention provides a diagnostic kit for liver cancer or a precancerous condition of the liver, comprising the antibody specific to the polypeptide comprising the amino acid sequence of SEQ ID NO: 1.

Furthermore, the present invention provides a polynucleotide coding for a polypeptide comprising the amino acid sequence of SEQ ID NO: 1 as well as a polynucleotide coding for a polypeptide having at least 80% homology to the amino acid sequence of SEQ ID NO: 1; having an amino acid sequence, at least an amino acid residue at position 39, 84, 296, or 300 from the N-terminus is Ala, an amino acid residue at position 85 is Thr, an amino acid residue at position 163 or 333 is Ser, an amino acid residue at position 195 or 257 is Leu, an amino acid residue at position 271 is Arg, an amino acid residue at position 297 is Asp, an amino acid residue at position 313 is Pro, or an amino acid residue at position 316 is Val, in correspondence with the amino acid sequence of SEQ ID NO: 1; and having immunogenicity inducing the production of an antibody against the polypeptide comprising the amino acid sequence of SEQ ID NO: 1.

Moreover, the present invention provides a polynucleotide coding for a polypeptide fragment having a partial sequence of the amino acid sequence of SEQ ID NO: 1; or a partial sequence of an amino acid sequence having at least 80% homology to the amino acid sequence of SEQ ID NO: 1, wherein at least an amino acid residue at position 39, 84, 296, or 300 from the N-terminus is Ala, an amino acid residue at position 85 is Thr, an amino acid residue at position 163 or 333 is Ser, an amino acid residue at position 195 or 257 is Leu, an amino acid residue at position 271 is Arg, an amino acid residue at position 297 is Asp, an amino acid residue at position 313 is Pro, or an amino acid residue at position 316 is Val, in correspondence with the amino acid sequence of SEQ ID NO: 1; and having immunogenicity inducing the production of an antibody against the polypeptide comprising the amino acid sequence of SEQ ID NO: 1.

Moreover, the present invention provides a polynucleotide comprising a nucleotide sequence from positions 436 to 1413 of SEQ ID NO: 2. These polynucleotides of the present invention include those isolated.

Moreover, the present invention provides a vector comprising any of the polynucleotides and a host cell transfected with the vector.

Furthermore, the present invention provides an RNA molecule with 15 to 25 nucleotide pairs, consisting of a nucleotide sequence corresponding to a partial sequence of a nucleotide sequence from positions 436 to 1413 of SEQ ID NO: 2, or a mutant nucleotide sequence of the nucleotide sequence with the addition, deletion, or substitution of at least one base, and suppressing the expression of a protein specific to human liver cancer.

Moreover, the present invention provides an RNA molecule consisting of the nucleotide sequence of SEQ ID NO: 3 as well as an RNA molecule consisting of a mutant nucleotide sequence of the nucleotide sequence of SEQ ID NO: 3 with the addition, deletion, or substitution of at least one base, and suppressing the expression of a protein specific to human liver cancer.

Moreover, the present invention provides a pharmaceutical composition that suppresses the expression of a protein specific to human liver cancer, comprising any of the RNA molecules.

Moreover, the present invention provides a method for the production of a knockout cell, comprising introducing any of the RNA molecules to a cell expressing a protein specific to human liver cancer or a mutant protein thereof, and maintaining the cell under a condition bringing about RNA interference by the RNA molecule, to degrade mRNA transcribed from a gene coding for the specific protein or the mutant protein thereof.

Moreover, the present invention provides a kit for the functional analysis of a protein specific to human liver cancer, comprising any of the RNA molecules.

According to the present invention, a novel polypeptides specific to liver cancer coded for the human proto-oncogene Pim-3, an antibody specific thereto, a polynucleotide coding for the specific polypeptide, and siRNA capable of specifically suppressing the expression of the specific polypeptide and being applied to the treatment of liver cancer are provided. As a result, the present invention made it possible to develop a novel diagnostic method, diagnostic reagent, and therapeutic method for liver cancer.

### Brief Description of the Drawings

Figure 1 is a graph showing the expression level of Pim-3 in an HBsTg mouse;
Figure 2 shows the polynucleotide sequence of hPim-3 and its corresponding polypeptide sequence;
Figure 3 is a diagram where the amino acid sequences of Pim-3 are compared among a human, a mouse, and a rat;
Figure 4 is a diagram where amino acid sequences are compared among human Pim-1, Pim-2, and Pim-3;
Figure 5 is a diagram showing hPim-3 and GAPDH mRNA expression in human normal tissue;
Figure 6 is a diagram showing human Pim-3 mRNA expression in a human liver cancer cell line;
Figure 7 is a diagram specifically showing hPim-3 expression in human liver cancer tissue;
Figure 8 is a graph showing a decrease in the survival activity of a human liver cancer cell, which was transfected with the siRNA of hPim-3;
Figure 9 is a diagram showing that cells started to exfoliate in the treated group on the 4th day and subsequent days of transfection of the siRNA of hPim-3; and
Figure 10 is a graph showing changes in the cell cycle of a human liver cancer cell in which the siRNA of Pim-3 was introduced.

### Most Preferable Mode for Carrying Out the Invention

A polypeptide specific to liver cancer of the present invention is a polypeptide comprising the amino acid sequence of SEQ ID NO: 1. The polypeptide of the present invention includes a homologue (polypeptide) having antigenicity in common with the polypeptide or a peptide fragment having antigenicity in common with them. The detection of the polypeptide of the present invention allows for the diagnosis of liver cancer. Moreover, the polypeptide, the homologue thereof, and the polypeptide fragment, of the present invention are useful in creating diagnostic and therapeutic antibodies for liver cancer. The steps of finding and isolating the specific polypeptide of the present invention are described in detail in Examples below.

The term "polypeptide" used herein means a primary structure that is an amino acid sequence, a polypeptide fragment with the primary structure, a polypeptide having biological activity accompanied with a three-dimensional structure, or a protein.

The term "homologue" used herein refers to a polypeptide having homology in an amino acid sequence to a polypeptide or protein with a particular amino acid sequence and having biological activity or antigenicity in common with the polypeptide or protein.

The term "siRNA" in the present invention means a short RNA fragment that suppresses the expression of a particular gene or a double-stranded RNA molecule that consists of the RNA fragment and its complementary strand.

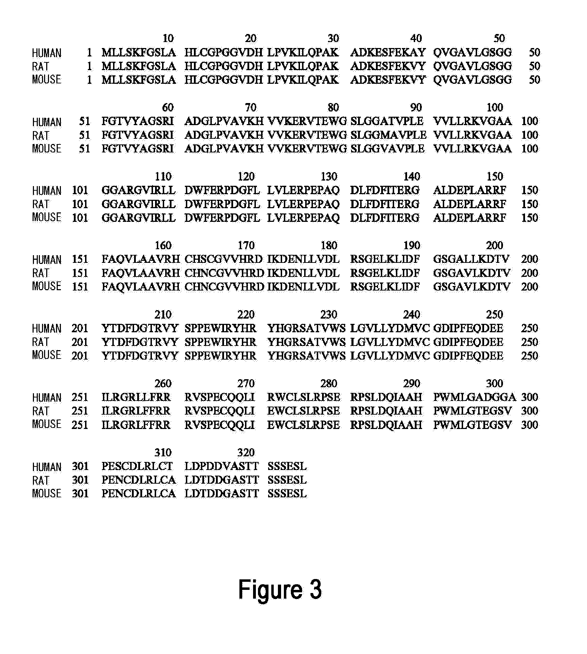

As shown in Figure 3, the amino acid sequence (SEQ ID NO: 1) of a human Pim-3 protein, the polypeptide specific to liver cancer of the present invention, has the following characteristic points of difference, when compared with those of rat and mouse Pim-3 proteins: the amino acid sequence of SEQ ID NO: 1 differs from those of the rat and mouse Pim-3 proteins, in that the amino acid residues at positions 39, 84, 296, and 300 from the N-terminus are Ala, the amino acid residue at position 85 is Thr, the amino acid residues at positions 163 and 333 are Ser, the amino acid residues at position 195 and 257 are Leu, the amino acid residue at position 271 is Arg, the amino acid residue at position 297 is Asp, the amino acid residue at 313 is Pro, and the amino acid residue at position 316 is Val.

Thus, the polypeptide homologue of the present invention is a polypeptide having at least 80% homology to the amino acid sequence of SEQ ID NO: 1; having an amino acid sequence, wherein at least an amino acid residue at position 39, 84, 296, or 300 from the N-terminus is Ala, an amino acid residue at position 85 is Thr, an amino acid residue at position 163 or 333 is Ser, an amino acid residue at position 195 or 257 is Leu, an amino acid residue at position 271 is Arg, an amino acid residue at position 297 is Asp, an amino acid residue at position 313 is Pro, or an amino acid residue at position 316 is Val, in correspondence with the amino acid sequence of SEQ ID NO: 1; and having immunogenicity inducing the production of an antibody against a polypeptide comprising the amino acid sequence of SEQ ID NO: 1. The homology of the homologue is especially 90%, preferably 95%, particularly preferably 97%.

The peptide fragment of the specific polypeptide of the present invention is a polypeptide fragment having a partial sequence of the amino acid sequence of SEQ ID NO: 1; or a partial sequence of an amino acid sequence having at least 80% homology to the amino acid sequence of SEQ ID NO: 1, wherein at least an amino acid residue at position 39, 84, 296, or 300 from the N-terminus is Ala, an amino acid residue at position 85 is Thr, an amino acid residue at position 163 or 333 is Ser, an amino acid residue at position 195 or 257 is Leu, an amino acid residue at position 271 is Arg, an amino acid residue at position 297 is Asp, an amino acid residue at position 313 is Pro, or an amino acid residue at position 316 is Val, in correspondence with the amino acid sequence of SEQ ID NO: 1; and having immunogenicity inducing the production of an antibody against the polypeptide comprising the amino acid sequence of SEQ ID NO: 1.

The homology of amino acid sequences used herein refers to identity (%) between a reference amino acid sequence and an amino acid sequence compared therewith when the sequences are aligned. The homology can be calculated using default (initial setting) parameters in analytical software such as BLAST (J. Mol. Biol., 215, 403 (1990)) and FASTA (Methods in Enzymology, 183, 63-69) that are standard programs for performing the homology search of nucleotide sequences and amino acid sequences.

In the present specification, the N-terminuses (amino terminuses) of the polynucleotide, the homologue, and the polypeptide fragment (hereinafter, abbreviated to the polynucleotide and so on, if necessary) are indicated in the left, and the C terminuses (carboxyl terminuses) thereof are indicated in the right, in accordance with the standard notation. The polypeptide of the present invention can have a carboxyl group (-COOH), carboxylate (-COO-), amide (-CONH₂), or ester (-COOR) at the C terminus. Examples of the side chain R of this ester include: C₁ to C₆ alkyl groups such as methyl, ethyl, n-propyl, isopropyl, and n-butyl; C₃ to C₈ cycloalkyl groups such as cyclopentyl and cyclohexyl; C₆ to C₁₂ aryl groups such as phenyl and α-naphthyl; and alkyl groups such as phenyl-C₁ to -C₂ alkyl groups such as benzyl and phenethyl or α-naphthyl-C₁ to -C₂ alkyl groups such as α-naphthylmethyl.

The polypeptide of the present invention also includes: those in which an amino group of an N-terminal amino acid residue is protected with a protecting group such as a formyl group and an acetyl group; those in which an N-terminal glutamine residue generated in vivo by cleavage is converted to pyroglutamic acid; those in which a substituent (e.g., -OH, -SH, an amino group, an imidazole group, an indole group, or a guanidino group) on the side chain of an intramolecular amino acid is protected with a suitable protecting group; or a conjugated protein such as so-called glycoprotein bound with a sugar chain.

The peptide fragment of the present invention is not particularly limited as long as it induces the production of an antibody against the polypeptide comprising the amino acid sequence of SEQ ID NO: 1. However, the peptide fragment of the present invention is a partial peptide of the polypeptide and the homologue thereof and has 20 to 200, preferably 20 to 100, more preferably 20 to 70 of the amino acids of their amino acid sequences.

Moreover, the polypeptide of the present invention can be used as a physiologically acceptable inorganic or organic acid-addition salt. Examples of the inorganic acid-addition salt include salts of hydrochloric acid, phosphoric acid, hydrobromic acid, and sulfuric acid, and examples of the organic acid-addition salt include salts of acetic acid, formic acid, propionic acid, fumaric acid, maleic acid, succinic acid, tartaric acid, citric acid, malic acid, oxalic acid, benzoic acid, methanesulfonic acid, and benzenesulfonic acid.

A composition of the present invention used for the production of an antibody specific to the polypeptide comprising the amino acid sequence of SEQ ID NO: 1 comprises the polypeptide, the homologue thereof, their peptide fragments, or their acid-addition salts. The composition may optionally contain ingredients such as a vehicle, a diluent, and an adjuvant.

An antibody of the present invention is an antibody that specifically reacts with the polypeptide comprising the amino acid sequence of SEQ ID NO: 1 or the peptide fragment thereof, which may be any of polyclonal and monoclonal antibodies.

The monoclonal antibody of the present invention can be produced by the following procedures: at first, the composition for producing the antibody is administered to non-human mammals. A complete Freund's adjuvant or incomplete Freund's adjuvant may be administered for enhancing the ability of the mammals to produce antibodies. The administration is typically performed once every 2 to 6 weeks with a total of approximately 2 to 10 doses. Examples of the non-human mammals include monkeys, rabbits, dogs, guinea pigs, mice, rats, sheep, goats, and chickens, with mice and rats generally preferred.

An individual in which antibody production has been observed is selected from the non-human mammals immunized with the antigen. Two to five days after final immunization, antibody-producing cells contained in the spleen or lymph node are collected from the individual and fused with myeloma cells from animals of the same or different species to thereby create monoclonal antibody-producing hybridomas. Antibody titer in antiserum is measured by allowing a labeled protein to react with the antiserum and then measuring the activity of the labeling agent bound with the antibody. Cell fusion can be performed by a routine method such as the method of Kohler and Milstein (Nature, 256, 495, 1975). For example, polyethylene glycol (PEG) or a Sendai virus can be used as a fusion promoter.

Examples of the myeloma cells include non-human mammalian myeloma cells such as NS-1, P3U1, SP2/O, and AP1, with P3U1 particularly preferred. A preferable ratio of the number of the antibody-producing cells (spleen cells) to the number of the myeloma cells, used in the fusion, is approximately 1:1 to 20. The spleen and myeloma cells can be fused by incubation at 20 to 40°C, preferably 30 to 37°C, for 1 to 10 minutes, with PEG (preferably PEG1000 to PEG6000) added at a concentration of approximately 10 to 80%.

The monoclonal antibody-producing hybridomas can be screened by a variety of methods. Examples of the methods include: a method that involves bringing a hybridoma culture supernatant into contact with a solid phase (e.g., a microplate) where an antigen is adsorbed either directly or in combination with a carrier and subsequently bringing a solution containing an anti-immunoglobulin antibody labeled with a radioactive substance, an enzyme, or the like, into contact therewith to detect the monoclonal antibody bound to the solid phase; and a method that involves bringing a hybridoma culture supernatant into contact with a solid phase where an anti-immunoglobulin antibody or the like is adsorbed and subsequently bringing a solution of a protein labeled with a radioactive substance, an enzyme, or the like, into contact therewith to detect the monoclonal antibody bound to the solid phase.

The monoclonal antibody-producing hybridomas can be selected by a routine method. For example, a medium for animal cells supplemented with HAT (hypoxanthine-aminopterin -thymidine), an RPMI 1640 medium containing 10 to 20% fetal bovine serum, a GIT medium containing 1 to 10% fetal bovine serum (Wako Pure Chemical Industries), or a serum-free medium for culturing hybridomas (SFM-101; Nissui Pharmaceutical) can be used. A culture temperature is 20 to 40°C, and a culture period is typically 5 days to 3 weeks. The culture can typically be performed under 5% carbonic acid gas. Antibody titer in the hybridoma culture supernatant can be measured in the same way as in the above-described measurement of antibody titer in antiserum.

The obtained monoclonal antibody can be separated and purified by a routine method such as: an immunoglobulin separation and purification method such as salting-out, alcohol precipitation, isoelectric precipitation, and electrophoresis; an absorption and desorption method with an ion exchanger (DEAE); an ultracentrifugation method; a gel filtration method; an antigen-bound solid phase; or a specific purification method performed by collecting only an antibody with a protein A active adsorbent and then dissociating the bond to obtain the antibody.

Alternatively, the polyclonal antibody of the present invention can be produced by immunizing non-human mammals with the polypeptide serving as an immunizing antigen or a complex of the peptide fragment thereof with a carrier protein and then collecting a antibody-containing component such as serum therefrom, followed by antibody separation and purification.

The mixing ratio of the immunizing antigen to the carrier protein is determined so that the antibody can efficiently be raised against the hapten that has been crosslinked with the carrier and used in immunization. For example, approximately 0.1 to 20 parts by weight, preferably approximately 1 to 5 parts by weight, of bovine serum albumin, bovine thyroglobulin, or hemocyanin can be used with respect to 1 part by weight of the hapten. For example, glutaraldehyde, carbodiimide, maleimide-activated ester, and activated ester reagents containing a thiol or dithiopyridyl group can be employed in the coupling between the hapten and the carrier.

The complex antigen may be administered alone or in combination with a carrier or diluent, or alternatively, for enhancing the ability of the mammals to produce antibodies, with a complete Freund's adjuvant or incomplete Freund's adjuvant. The administration is typically performed once every approximately 2 to 6 weeks with a total of approximately 3 to 10 doses. The polyclonal antibody is collected from the blood, ascites, yolk, or the like, of the immunized mammal. Polyclonal antibody titer in antiserum can be measured in the same way as in the above-described measurement of antibody titer in antiserum. The polyclonal antibody can be separated and purified in the same way as in the above-described separation and purification of the monoclonal antibody.

A diagnostic kit for liver cancer or a precancerous condition of the liver of the present invention comprises the polyclonal or monoclonal antibody thus obtained. The diagnostic kit optionally contains wells for immune reaction, a staining agent, an enzyme-labeled antibody for detection, a cleansing liquid, an antibody diluent, a sample diluent, an enzyme substrate, a diluent of an enzyme substrate solution, and additional reagents.

A polynucleotide of the present invention is a polynucleotide that codes for the polypeptide comprising the amino acid sequence of SEQ ID NO: 1, the homologue (polypeptide) having antigenicity in common with the polypeptide, or the peptide fragment having antigenicity in common with them. Especially, a polynucleotide comprising a nucleotide sequence from positions 436 to 1413 of SEQ ID NO: 2 is preferable.

The polynucleotide of the preset invention may be any of DNA and RNA. The DNA may be in any form of genomic DNA, a genomic DNA library, cDNA derived from the liver cancer cell or tissue, and synthetic DNA. Moreover, examples of a vector used in the library include a bacteriophage, a plasmid, a cosmid, or a phagemid. The polynucleotide of the present invention may be any of those amplified directly by an RT-PCR method using total RNA or an mRNA fraction prepared from the liver cancer cell or tissue.

The polynucleotide of the present invention includes a polynucleotide that hybridizes under stringent conditions to the polynucleotide comprising a nucleotide sequence from positions 436 to 1413 of SEQ ID NO: 2 or a polynucleotide complementary to the polynucleotide, and codes for the polypeptide having antigenicity in common with the polypeptide comprising the amino acid sequence of SEQ ID NO: 1.

The hybridization can be performed by a routine method described in, for example, Molecular Cloning, 2nd ed., J. Sambrook et al., Cold Spring Harbor Lab. Press, 1989.
The stringent conditions refer to, for example, conditions under which a sodium concentration is approximately 19 to 40 mM, preferably approximately 19 to 20 mM, and a temperature is approximately 50 to 70°C, preferably approximately 60 to 65°C.

DNA coding for the polypeptide of the present invention can be selected by amplification by a PCR method using a synthetic DNA primer having a portion of a nucleotide sequence coding for the polypeptide of the present invention, or otherwise, by hybridization between the DNA incorporated in an appropriate vector and a labeled DNA fragment or synthetic DNA coding for a partial or entire region of the polypeptide of the present invention. The hybridization method can be performed by a method described in, for example, Molecular Cloning, 2nd ed., J. Sambrook et al., Cold Spring Harbor Lab. Press, 1989. The obtained DNA can be used, for each purpose, either directly or after digestion with a restriction enzyme or the addition of a linker thereto. The DNA may undergo the addition of ATG serving as a translation initiation codon to the 5'-terminal side and the addition of TAA, TGA, or TAG serving as a translation termination codon to the 3'-terminal side. These translation initiation and termination codons can also be added thereto by use of an appropriate synthetic DNA adaptor.

In this context, the primer used is a PCR primer comprising at least 15 nucleotides corresponding to the polynucleotide coding for the polypeptide comprising the amino acid sequence of SEQ ID NO: 1 or corresponding to the polynucleotide complementary thereto.

Moreover, these primers can be used to make a kit that detects the polynucleotide coding for the polypeptide comprising the amino acid sequence of SEQ ID NO: 1.

An expression vector for the polypeptide of the present invention can be produced by cutting a DNA fragment of interest from the DNA coding for the polypeptide of the present invention and ligating the DNA fragment to a region downstream from a promoter in an appropriate vector.

In this context, examples of the vector used include plasmids derived from *E. coli* (e.g., pBR322, pBR325, pUC12, and pUC13), plasmids derived from *Bacillus subtilis* (e.g., pUB 110, pTP5, and pC194), plasmids derived from yeasts (e.g., pSH19 and pSH15), bacteriophages such as λ-phages, and animal viruses such as retroviruses, vaccinia viruses, and baculoviruses, in addition to pA1-11, pXT1, pRc/CMV, pRc/RSV, and pcDNAI/Neo.

The promoter used in the present invention may be any suitable promoter that is compatible with a host used. When an animal cell is used as the host, examples of the promoter include SRα promoters, SV40 promoters, LTR promoters, CMV promoters, and HSV-TK promoters. Alternatively, examples of the promoter include: trp promoters, lac promoters, recA promoters, λPL promoters, lpp promoters, and T7 promoters when the host is a bacterium of the genus *Escherichia;* SPO 1 promoters, SPO2 promoters, and penP promoters when the host is a bacterium of the genus *Bacillus;* PHO5 promoters, PGK promoters, GAP promoters, and ADH promoters when the host is a yeast; and polyhedron promoters and P10 promoters when the host is an insect cell.

The expression vector of the present invention that can be used can further contain an enhancer, a splicing signal, a poly A addition signal, a selective marker, an SV40 replication origin, and so on, if desired. Examples of the selective marker include a dihydrofolate reductase gene, an ampicillin resistance gene, and a neomycin resistance gene. If necessary, a signal sequence suitable for a host is added to the N-terminal side of the polypeptide of the present invention. Examples of the signal sequence that can be utilized include: a PhoA signal sequence and an OmpA signal sequence when the host is a bacterium of the genus *Escherichia*; an α-amylase signal sequence and a subtilisin signal sequence when the host is a bacterium of the genus *Bacillus;* an MFα signal sequence and an SUC2 signal sequence when the host is a yeast; and an insulin signal sequence, an α-interferon signal sequence, and an antibody molecule signal sequence when the host is an animal cell. The vector thus constructed, which contains the DNA coding for the polypeptide of the present invention, can be used to manufacture a transformant.

Examples of the host used in the present invention include a bacterium of the genus *Escherichia,* a bacterium of the genus *Bacillus,* a yeast, an insect cell, an insect, and an animal cell.

The bacterium of the genus *Bacillus* is transformed by a method described in, for example, Molecular & General Genetics, Vol. 168, 111, (1979). The yeast is transformed by a method described in, for example, Methods in Enzymology, Vol. 194, 182-187, (1991) or Proc. Natl. Acad. Sci. USA, Vol. 75, 1929, (1978). The insect cell or the insect is transformed by a method described in, for example, Bio/Technology, 6, 47-55, (1988). The animal cell is transformed by a method described in, for example, Cell Technology Suppl. 8, New Experimental Protocol in Cell Technology, 263-267,(1995) (issued by Shujunsha) or Virology, Vol. 52, 456, (1973). The transformant transformed with the expression vector containing the DNA coding for the polypeptide of the present invention can be created in this way.

An appropriate medium used for culturing the transformant from the bacterium of the genus *Escherichia* or *Bacillus* used as the host is a liquid medium, which may contain a carbon source, a nitrogen source, inorganic matter, and so on, necessary for the growth of the transformant. In this context, examples of the carbon source include glucose, dextrin, soluble starch, and sucrose. Examples of the nitrogen source include ammonium salts, nitrates, com steep liquor, peptone, casein, meat extracts, soybean cake and potato extracts. Examples of an inorganic nutrient include calcium chloride, sodium dihydrogen phosphate, and magnesium chloride. For example, a yeast extract, vitamins, and a growth promoter may further be added thereto. Desirably, the pH of the medium is approximately 5 to 8.

Preferably, a medium for culturing the bacterium of the genus *Escherichia* is, for example, an M9 medium containing glucose and casamino acid (Miller, Journal of Experiments in Molecular Genetics, 431-433, Cold Spring Harbor Laboratory, New York, 1972).

A drug such as 3β-indolylacrylic acid can be added to the medium in order to allow the promoter to efficiently work. When the bacterium of the genus *Escherichia* is used as the host, culture is typically performed at approximately 15 to 43°C for approximately 3 to 24 hours and can be performed optionally with aeration or stirring. When the bacterium of the genus *Bacillus* is used as the host, culture is typically performed at approximately 30 to 40°C for approximately 6 to 24 hours and can be performed optionally with aeration or stirring.

A medium for culturing the transformant from the yeast used as the host is, for example, a Burkholder minimum medium (Bostian, K. L. et al., Proc. Natl. Acad. Sci. USA, Vol. 77, 4505, (1980)) or an SD medium containing 0.5% casamino acid (Bitter, G.
A. et al., Proc. Natl. Acad. Sci. USA, Vol. 81, 5330, (1984)). It is preferred that the pH of the medium should be adjusted to approximately 5 to 8. Culture is typically performed at approximately 20 to 35°C for approximately 24 to 72 hours, optionally with aeration or stirring.

A medium employed for culturing the transformant from the insect cell used as the host is, for example, Grace's Insect Medium (Grace, T.C.C., Nature, 195, 788, (1962)) supplemented with an additive such as inactivated 10% bovine serum. It is preferred that the pH of the medium should be adjusted to approximately 6.2 to 6.4. Culture is typically performed at approximately 27°C for approximately 3 to 5 days, optionally with aeration or stirring.

A medium employed for culturing the transformant from the animal cell used as the host is, for example, an MEM medium containing approximately 5 to 20% fetal bovine serum (Science, Vol. 122, 501, (1952)), a DMEM medium (Virology, Vol. 8, 396, (1959)), an RPMI1640 medium (The Journal of the American Medical Association Vol. 199, 519, (1967)), or a 199 medium (Proceeding of the Society for the Biological Medicine, Vol. 73, 1, (1950)). Preferably, the pH of the medium is approximately 6 to 8. Culture is typically performed at approximately 30 to 40°C for approximately 15 to 60 hours, optionally with aeration or stirring. In this way, the polypeptide of the present invention can be produced within the cell, in the cell membrane, or without the cell, of the transformant.

The polypeptide of the present invention is separated and purified from the resulting culture according to a routine method. For example, the polypeptide of the present invention is extracted from the cultured bacterium or cell by procedures in which the bacterium or cell that has been cultured is collected by a method known in the art and suspended in an appropriate buffer solution, and the resulting bacterium or cell is then ruptured by sonication, lysozyme, and/or freezing and thawing, followed by centrifugation or filtration to thereby give a crude polypeptide extract. The buffer solution may contain a denaturant such as urea and guanidine hydrochloride, and a surfactant such as TritonX-100™. When the polypeptide is secreted into the culture solution, the bacterium or the cell is separated from a supernatant by a method known in the art after the completion of culture to collect the resulting supernatant. The polypeptide contained in the culture supernatant or the extract thus obtained is purified by appropriately combined separation and purification methods known in the art. Examples of these separation and purification methods known in the art include: a method that utilizes solubility such as salting-out and solvent precipitation; a method that mainly utilizes a difference in molecular weight such as dialysis, ultrafiltration, gel filtration, and SDS-polyacrylamide gel electrophoresis; a method that utilizes a difference in electric charge such as ion-exchange chromatography; a method that utilizes specific affinity such as affinity chromatography; a method that utilizes a difference in hydrophobicity such as reverse-phase high performance liquid chromatography; and a method that utilizes a difference in isoelectric point such as isoelectric focusing.

When the polypeptide is obtained in a free form, the polypeptide can be converted into a salt by a method known in the art, whereas when the polypeptide is obtained in a salt form, the polypeptide can be converted into a free form or other salts by a method known in the art. Before or after polypeptide purification, an appropriate protein modification enzyme is allowed to act on the polypeptide produced by the recombinant to thereby provide for the addition of arbitrary modification thereto or the partial removal of the polypeptide. Examples of the protein modification enzyme include trypsin, chymotrypsin, arginyl endopeptidase, protein kinase, and glycosidase. The polypeptide of the present invention thus generated can be confirmed by, for example, enzyme immunoassay or western blotting using a specific antibody.

The present invention further provides an RNA molecule suppressing the expression of a protein specific to human liver cancer. The RNA molecule is considered to suppress gene expression by cleaving mRNA coding for the polypeptide comprising the amino acid sequence of SEQ ID NO: 1, at a site in its corresponding sequence. The RNA molecule appears to bring about the same phenomenon as RNA interference described in, for example, the paper of Fire et al. (Fire et al., Nature 391, 806-811, (1998)).

The RNA molecule is an RNA molecule consisting of a nucleotide sequence corresponding to a partial sequence of a nucleotide sequence from positions 436 to 1413 of SEQ ID NO: 2, or a mutant nucleotide sequence of the nucleotide sequence with the addition, deletion, or substitution of at least one base, and suppressing the expression of a protein specific to human liver cancer. The number of nucleotide pairs of the RNA molecule is 15 to 25, preferably 18 to 24, particularly preferably 21 to 23.

The specific protein is the polypeptide comprising the amino acid sequence of SEQ ID NO: 1 or a mutant polypeptide thereof. The mutant polypeptide is a polypeptide having an amino acid sequence having 80% homology, preferably 90% homology, particularly preferably 95% homology, to the amino acid sequence of SEQ ID NO: 1.

The RNA molecule of the present invention may be an RNA molecule consisting of the nucleotide sequence of SEQ ID NO: 3, or an RNA molecule consisting of a mutant nucleotide sequence of the nucleotide sequence of SEQ ID NO: 3 with the addition, deletion, or substitution of at least one base, and suppressing the expression of a protein specific to human liver cancer. The RNA molecule (siRNA) can be designed on the basis of the sequence of polynucleotide of the present invention according to a method known in the art (e.g., Nature, Vol. 411, pp. 494, 2001).

When the RNA molecule of the present invention is used in vivo or in vitro, it is used in the form of a double-stranded RNA molecule that consists of the RNA molecule and its complementary RNA. In this case, it is preferred that they should be treated so that the double-stranded RNA molecule is not degraded in a cell or is not dissociated into single strands. The treatment is performed by, for example, a method that adds a hydroxyl group to the 3' end of the RNA molecule, forms chemical bonds at both ends of the duplex through thiophosphoryl groups, or induces a chemical bond between both strands by ultraviolet radiation or through a bifunctional group such as bis(2-chloromethyl)amine, 4-thiouracil, or psoralen.

Since the RNA molecule of the present invention suppresses the expression of the protein specific to human liver cancer, the RNA molecule can be employed in the treatment of liver cancer and as a pharmaceutical composition that prevents a cell in a precancerous condition from progressing to cancer.

The RNA or a salt thereof can be administered orally or parenterally, and the parenteral administration includes local administration to tissue.

When a pharmaceutical composition of the present invention is orally administered, pharmaceutical ingredients such as carriers widely used, for example, fillers, expanders, binders, disintegrants, disintegration inhibitors, buffers, tonicity agents, emulsifiers, dispersants, stabilizers, coating agents, surfactants, absorption promoters, humectants, wetting agents, adsorbents, lubricants, and excipients can be used. Additives such as coloring agents, preservatives, perfumes, flavoring agents, and sweetening agents may optionally be used.

Concrete examples of the pharmaceutical ingredients include: excipients such as milk sugar, white sugar, sodium chloride, grape sugar, urea, starch, calcium carbonate, kaoline, crystalline cellulose, and silicic acid; binders such as water, ethanol, simple syrup, grape sugar fluids, starch fluids, gelatin solutions, carboxymethylcellulose, shellac, methylcellulose, potassium phosphate, and polyvinylpyrrolidone; disintegrants such as dried starch, sodium alginate, agar powder, laminaran powder, sodium hydrogencarbonate, calcium carbonate, polyoxyethylene sorbitan fatty acid ester, sodium lauryl sulfate, stearic acid monoglyceride, starch, and milk sugar; disintegration inhibitors such as white sugar, stearic acid, cacao butter, and hydrogenated oil; absorption promoters such as quaternary ammonium salts and sodium lauryl sulfate; humectants such as glycerin and starch; adsorbents such as starch, milk sugar, kaolin, bentonite, and colloidal silicic acid; and lubricants such as purified talc and stearate. In addition, the technique of a drug delivery system known in the art is optionally adopted for each of the dosage forms to provide for the sustained release, local application (e.g., troches, buccals, and sublingual tablets), drug release control, conversion to an enteric coated form, conversion to a gastrolytic form, and so on, of the pharmaceutical composition.

When the pharmaceutical composition of the present invention is parenterally administered, the pharmaceutical composition can be used in forms such as administration by injection such as instillation, intravenous injection, hypodermic injection, and intramuscular injection, and endorectal administration with suppositories, for example, suppositories made of fats and oils and water-soluble suppositories. The pharmaceutical composition in such a form can be prepared with ease by a routine method using a typical carrier in the pharmaceutical field.

The siRNA of the present invention is safe and low toxic and as such, can be administered to, for example, humans or other mammals (e.g., rats, mice, guinea pigs, rabbits, sheep, pigs, cattle, horses, cats, dogs, and monkeys). The amount of the siRNA or a salt thereof administered differs depending on the condition of liver cancer, objects to be administered, administration routes, and so on. For example, when orally administered, the siRNA or the salt thereof is generally administered at approximately 10 to 4000 mg, preferably approximately 20 to 2000 mg, more preferably approximately 50 to 500 mg, per day for an adult patient weighing 60 kg. When parenterally administered, it is preferred that the siRNA, for example in the form of injection, should be administered through the vein in an amount per dose of approximately 10 to 2000 mg, preferably approximately 20 to 1000 mg, more preferably approximately 50 to 500 mg, per day for an adult patient weighing 60 kg although the amount differs depending on objects to be administered, the condition of liver cancer, and so on.

A Pim-3 gene knockout cell can be produced by introducing the RNA molecule to a cell expressing a protein specific to human liver cancer or a mutant protein thereof, and maintaining the cell under a condition bringing about RNA interference by the RNA molecule, to degrade mRNA transcribed from a gene coding for the specific protein or the mutant protein thereof. The use of this knockout cell allows for the examination of the polypeptide coded for by the Pim-3 gene for its function. Thus, a kit for the functional analysis of the Pim-3 gene protein specific to human liver cancer can be created by utilizing the RNA molecule.

### Industrial Applicability

A novel polypeptide specific to liver cancer coded for by a human proto-oncogene Pim-3, an antibody specific thereto, a polynucleotide coding for the specific polypeptide, and siRNA specifically suppressing the expression of the specific polypeptide, which are provided by the present invention have applications such as a novel diagnostic method, diagnostic reagent, and therapeutic method for liver cancer.

### Example 1

### Preparation of liver cancer model mouse

An HBsAg transgenic mouse of lineage 107-5D (official designation: Tg [Alb-1, HBV] Bri66; inbred B10D2, H-2d) was provided by Dr. F. V. Chisari (The Scripps Research Institute, La Jolla, CA). The lineage 107-5D contains the entire HBV envelope coding region (subtype ayw) placed under the transcriptional control of an albumin promoter and expresses small, middle, and large HBV envelope proteins in its liver cells (*2).

This mouse is immunologically tolerant to HBs transgenes at the T cell level (*3) and therefore displays no symptom of liver disease during the life time unless receiving the adoptive transfer of cytotoxic T cells specific to HBsAg (*2, *4).

A model of liver disease associated with chronic hepatitis was prepared by the following procedures: a male HBsAg transgenic mouse was thymectomized and exposed to radiation (900 cGy), followed by bone marrow cell transplantation from a syngeneic nontransgenic B10D2 (H-2d) mouse to reconstitute the hematopoietic system of the transgenic mouse. One week after the bone marrow transplantation, the mouse was subjected to the transplantation of 108 spleen cells collected from a syngeneic nontransgenic B10D2 (H-2d) mouse that had intraperitoneally been infected 3 weeks earlier with a recombinant vaccinia virus (HBs-vac) that expressed HBsAg. Twelve to fifteen months after the lymphocyte transplantation, the mouse developed the complex foci of liver cancer (*1).

A non-tumor site and a tumor site were separately isolated by drawing a distinction between them under a stereoscopic microscope. Then, the obtained non-tumor site was observed under a light microscope to confirm the presence of liver cells that were non-cancerous cells but were in an abnormal form. This non-tumor site containing non-cancerous cells was used as a lesion in a precancerous condition in subsequence experiments. Moreover, a liver tissue control was treated with a phosphate buffer solution or obtained from a transgenic mouse to which unimmunized mouse spleen cells were transplanted.

### Example 2

### Screening of gene of protein specific to liver cancer

Total RNAs extracted from the liver tissues of the model and normal mice of Example 1 were subjected to fluorescent differential display (FDD) according to the method described by Ito et al (*5).

Namely, each total RNA was isolated using RNA-Bee (Biotecx Laboratories) and then treated with RNase-free DNase (Takara Shuzo). Reverse transcription reaction was performed using 2.5 µg of the purified RNA, reverse transcriptase (SuperScript reverse transcriptase; Invitrogen) and fluorescently labeled anchor primers GT15A, GT15C, and GT15G.

cDNA corresponding to 50 ng of the RNA was synthesized and used together with 0.5 µM anchor primers, 0.5 µM arbitrary primers (10 mer kit A; Operon), 50 µM distinct dNTPs, 1 unit of Gene Taq DNA polymerase (Nippon Gene), and 1 unit of Taq DNA polymerase (Takara Shuzo) to perform polymerase chain reaction (PCR). The resulting PCR product was separated on a 6% polyacrylamide-8 M urea gel and analyzed with Vistra Fluor Imager SI (Molecular Dynamics).

The band of interest was extracted from the gel and cloned using a pSTBlue-1 vector (Novagen). Then, the nucleotide sequence of the inserted cDNA was determined using CEQ 2000 DNA Analysis System (Beckman Coulter) and analyzed by BLAST program for comparing it with sequences in Gene Bank database.

In this way, the present inventors could compare gene expression patterns between the lesion in a precancerous condition and the normal liver by FDD using combinations of 60 independent primers. In 2 independent experiments, the expression of 38 and 56 bands was respectively increased or decreased with reproducibility. The nucleotide sequences of the obtained bands whose expression was increased or decreased in the lesion in a precancerous condition could be confirmed to have 24 and 19 independent genes, respectively. Previous research reports indicate that most of these genes are expressed in the liver.

However, there was no report of expression in normal liver cells and liver cancer cells as for the Pim-3 gene whose expression was increased in the lesion in a precancerous condition. Therefore, the present inventors conducted semiquantitative RT-PCR analysis by focusing on the Pim-3, that is, Pim3 belonging to proto-oncogene family with serine/threonine kinase activity, including Pim-1 and Pim-2.

### Example 3

### Semiquantitative RT-PCR analysis of Pim-3

The total RNA extracted by the method of Example 2 was used to perform semiquantitative PCR analysis according to the method described in the paper of Kitamura et al (*6). Amplification was performed using primer sets for amplifying Pim-3 genes and glyceraldehyde 3-phosphate dehydrogenase (GAPDH) genes to give corresponding cDNA.

The sequences of these primers are shown below.

Pim-3 Sense:
   5'-AAGCAGTGACCTCTGACCCCTGGTGACC-3' (SEQ ID NO: 3)
Pim-3 Antisense:
   5'-CAGCGGAACCGCTCATTGCCAATGG-3' (SEQ ID NO: 4)
GAPDH Sense:
   5'-ACCACAGTCCATGCCATCAC-3' (SEQ ID NO: 5)
GAPDH Antisense:
   5'-TCCACCACCCTGTTGCTGTA-3' (SEQ ID NO: 6)

cDNA was synthesized from 1 µg of the purified total RNA in 20 µl of the reaction solution containing reverse transcriptase (ReverTra Ace; Toyobo) and random hexanucleotide primers (Amersham Biotech). Gene amplification was performed using 0.5 µl of the obtained cDNA, Taq DNA polymerase (Takara Shuzo), and the primers combined between SEQ ID NOs: 3 and 4 or 5 and 6 in Gene Amp PCR System 9700 (Applied Biosystems) under the following temperature conditions: (94°C for 2 min), (94°C for 30 sec, 55°C for 1 min, and 72°C for 1 min)x30 cycles, followed by (72°C for 5 min).

The resulting PCR product was separated on 1.5% agarose gel and visualized by ethidium bromide staining. The intensity of bands was measured using NIH image analysis software Ver. 1.61 (National Institutes of Health, Bethesda, MD) to calculate the ratio to GAPDH.

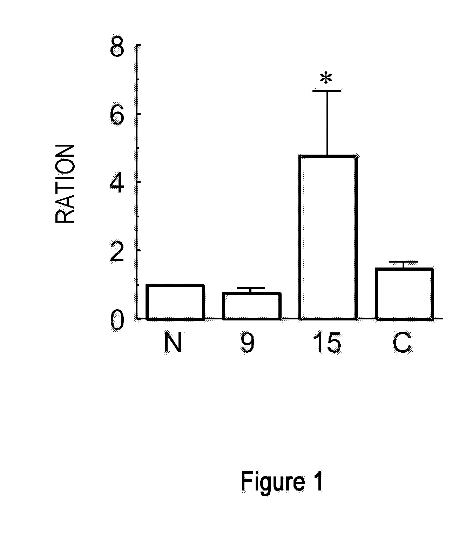

Figure 1 is a graph showing the expression level of Pim-3. In Figure 1, the reference character N denotes data measured by semi-quantitative RT-PCR analysis conducted on the total RNA extracted from the HBsTg mouse liver tissue before the introduction of immunized spleen cells; the reference character 9 denotes data (lesion of chronic hepatitis) measured in the same way 9 months after the introduction of immunized spleen cells; the reference character 15 denotes data (lesion in a precancerous condition) measured in the same way 15 months after the introduction of immunized spleen cells; and the reference character C denotes data measured in the same way 15 months after the introduction of unimmunized spleen cells. The ratio of the Pim-3 PCR product to the GAPDH PCR product was determined to calculate specific intensity on the assumption that the ratio for untreated mouse was 1.0. Then, an average and SD were calculated and shown in the drawing. Statistical significance was evaluated using the ANOVA test, and p<0.05 was defined as being statistically significant.

The result of this semiquantitative RT-PCR analysis focusing on Pim-3 is consistent with the result of the FDD analysis and indicates, as is obvious from Figure 1, that Pim-3 mRNA expression is significantly up-regulated in tissue in a precancerous condition, as compared with the control.

### Example 4

### Cloning and sequencing of human Pim-3 gene

For determining the nucleotide sequence of a human homologous gene of Pim-3 (hPim-3) judged in Examples 2 and 3 as being a gene specifically expressed in the precancerous condition of the mouse liver cancer model, a cDNA library was created from a human liver cancer cell line HepG2 and screened to perform the cloning and sequencing of Pim-3 cDNA.

At first, the cell line HepG2 was placed in a humid environment filled with air containing 5% carbonic acid gas and was cultured at 37°C in a Dulbecco's modified Eagle's medium (DMEM; Sigma) supplemented with 10% fetal bovine serum (Atlanta Biologicals, Norcross, Ga.) inactivated by heat.

Subsequently, total RNA was isolated from the cell line HepG2, and mRNA containing a poly A tail was separated with an mRNA purification kit (Oligotex™-dT30 mRNA Purification kit; Takara Shuzo). cDNA was synthesized from the obtained mRNA by use of reverse transcriptase (SuperScript reverse transcriptase; Invitrogen) and oligo-dT primers, and a cDNA library was constructed into pCMVSPORT6 (Invitrogen) using *E. coli* DH10B (Invitrogen) according to the instruction. Primary-stage screening was performed using GENE TRAPPER cDNA Positive Selection System (Invitrogen), with an oligomer of SEQ ID NO: 7 used as a probe.

Oligomer sequence: 5'-CTGTGAAGCACGTGGTGAAG-3' (SEQ ID NO: 7)

The obtained colonies were subjected to secondary-stage colony PCR screening using the primer sets described above.

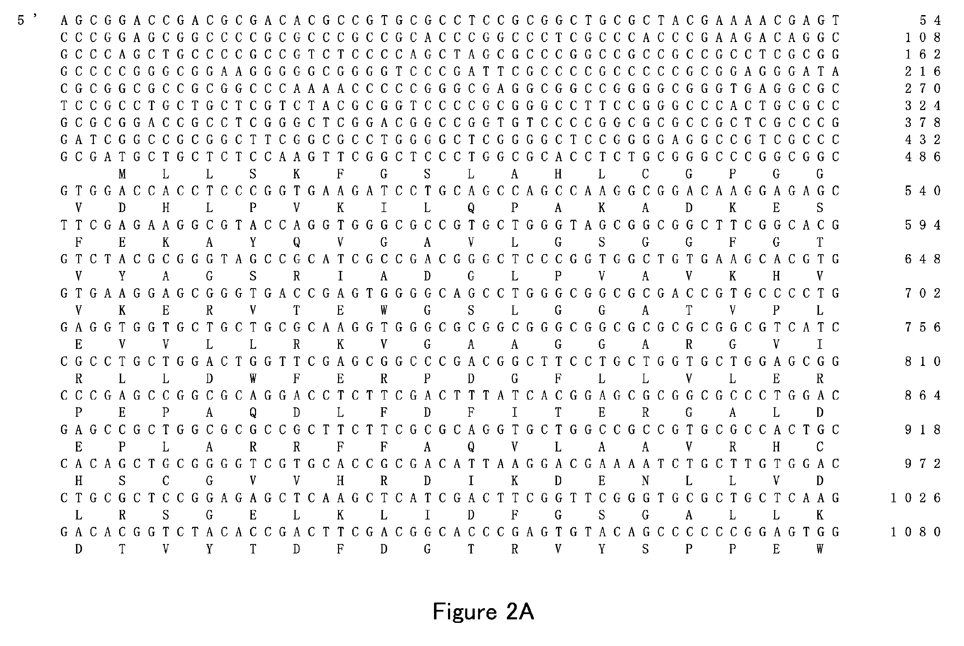

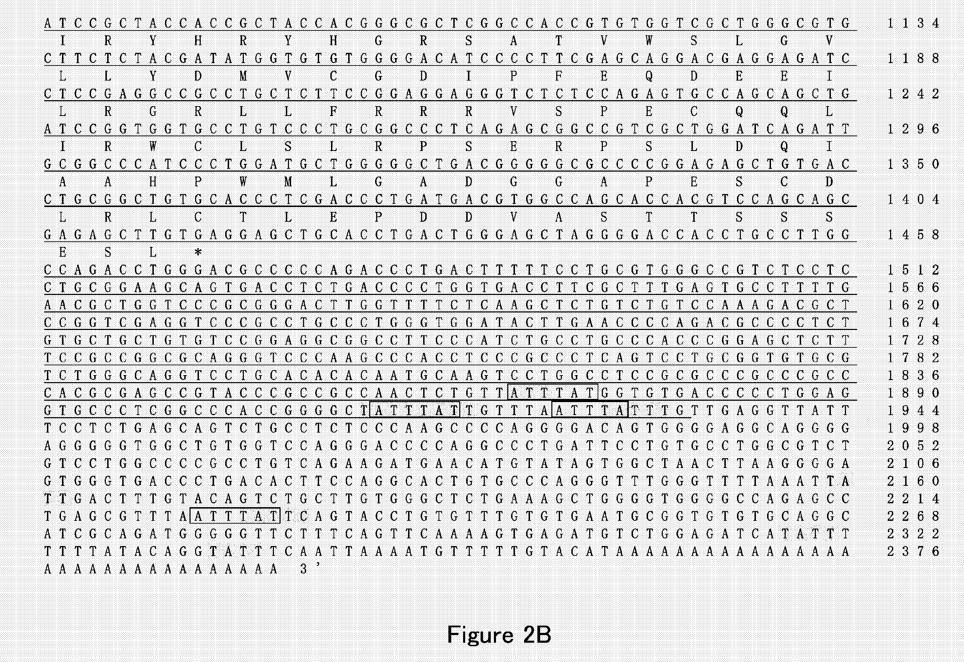

The secondary-stage screening yielded 3 positive clones, and these 3 independent cDNA clones contained the same insert consisting of 2,392-bp nucleotide sequence.
While 5' untranslated region has a GC content of 82.3%,3' untranslated region contained 5 copies of ATTTA motifs and 8 copies of TATT motifs (Figure 2). This sequence was identical to a particular partial sequence of the hPim-3 cDNA sequence shown on EST database. Its open reading frame (ORF) coded for an amino acid sequence with 326 residues (polypeptide (protein) with a molecular weight of 35, 875 comprising SEQ ID NO: 1) (Figure 3). In Figure 2, AT-rich motifs (TATT or ATTTA) were highlighted.

In addition, the amino acid sequence (SEQ ID NO: 1) obtained from the ORF had high identity (95.0%) to those of mouse and rat Pim-3 polynucleotides (Figure 3). Based on these results, the present inventors judged the clone human Pim-3 cDNA.

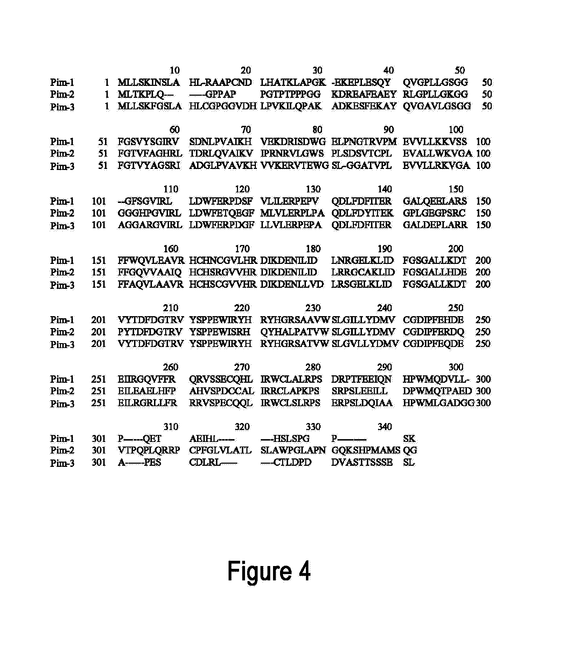

The human Pim-3 polypeptide coded for the cDNA exhibited high homology at the amino acid level to quail Pim (73.9%) and Xenopus Pim-3 (Pim-1: 68.7%) polypeptides. In addition, the human Pim-3 exhibited high homology at the amino acid level to human Pim-1 (57.1%) and human Pim-2 (44.0%) polypeptides (Figure 4). The comparison of the Pim-3 among a human, a rat, and a mouse in Figure 3 as well as the comparison of amino acid sequences among human Pim-1 to Pim-3 in Figure 4 were performed using DNASIS-Mac version 3.0 software (Hitachi Software engineering Co., Ltd., Yokohama, Japan). The same amino acid residues in these sequences as in hPim-3 were highlighted.

### Example 5

### Expression of human Pim-3 mRNA in human normal tissue

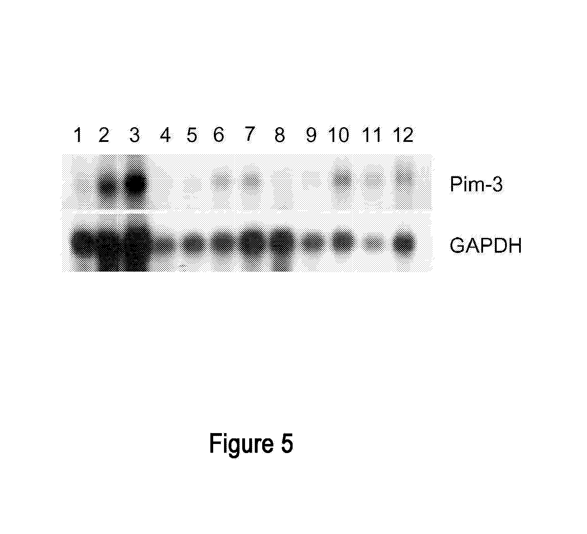

For examining the expression of hPim-3 mRNA in human normal tissue, the expression of 2.4-kbp mRNA corresponding to the hPim-3-positive cDNA clone (2,392 bp) of Example 3 was examined by northern blot analysis. Tissues to be examined were 1: brain, 2: heart, 3: skeletal muscle, 4: colon, 5: thymus, 6: spleen, 7: kidney, 8: liver, 9: small intestine, 10: placenta, 11: lung, and 12: peripheral blood leukocyte (the numbers are lane reference numerals in Figure 5). For evaluating mRNA levels, GAPDH mRNA expression in the same tissues was analyzed and shown in parallel to the respective hPim-3 lanes.

The analysis of mRNA by the northern blot analysis was performed using Human 12-Lane MTNTM Blot (Clontech, Palo Alto, CA). The mRNA was transcribed in vitro, to which a digoxigenin (DIG)-labeled probe was then hybridized at each appropriate temperature (70°C for Pim-3 and 68°C for GAPDH). Then, the blot was washed at 68°C for 15 minutes using a 2xSSC buffer containing 0.1% sodium dodecyl sulfate (SDS) and subsequently at 68°C for 15 minutes using a 0.5xSSC buffer containing 0.1% SDS. The hybridized probe was detected with a DIG detection kit (Boehringer Mannheim Biochemicals) according to the instruction.

Consequently, as shown in Figure 5, Pim-3 was expressed strongly in the heart and skeletal muscle and moderately in the brain, spleen, kidney, placenta, lung, and peripheral blood leukocyte, whereas the expression of Pim-3 was not detected in the colon, thymus, liver, and small intestine.

### Example 6

### Expression of human Pim-3 mRNA in human liver cancer cell lines

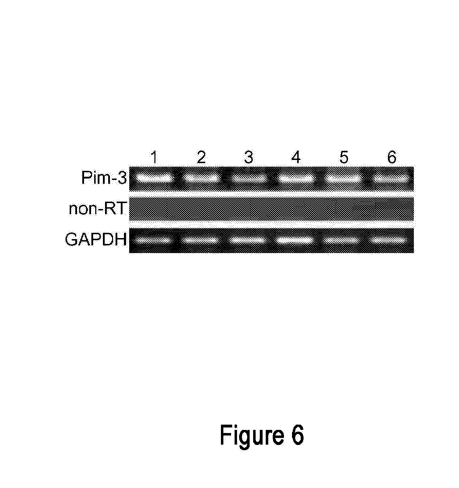

For examining the expression of hPim-3 mRNA in human liver cancer cells, RT-PCR analysis was performed. Namely, total RNA was extracted by the method of Example 2 from human liver cancer cell lines HepG2 (1), Hep3B (2), HLE (3), HLF (4), HuH7 (5), and SK-Hep (6) and subjected to RT-PCR using primer sets for amplifying Pim-3 genes and glyceraldehyde 3-phosphate dehydrogenase (GAPDH) genes. As a result, as shown in Figure 6, hPim-3 was detected in all the liver cancer cells analyzed. The numbers (1) to (6) described above are reference numerals in Figure 6.

### Example 7

### Preparation of antibody against Pim-3 polypeptide

An anti-Pim-3 antibody was custom-made by Asahi Techno Glass. At first, a peptide fragment corresponding to amino acid residues from the positions 13 to 32 of the amino acid sequence (SEQ ID NO: 1) of the Pim-3 polypeptide was bound with keyhole limpet hemocyanin (KLH) to create a compound (KLH: CGPGGVDHLPVKILQPAKAD: SEQ ID NO: 8), which was in turn administered to 2 chickens for immunization. The yolk was obtained both before and after immunization, and IgY proteins thereof were purified using EGGstract IgY Purification System (Promega) according to the instruction and quantified by measuring absorbance at 280 nm.

### Example 8

### Expression of hPim-3 in human liver cancer tissue

For examining the expression of hPim-3 in human liver cancer tissue, immunohistochemical analysis using the antibody was performed in human liver cancer tissue and the normal liver.

At first, tissue of a human liver cancer area embedded in paraffin was deparraffinized in xylene and treated with a gentle gradient with an ethanol concentration (100 to 70%) to perform rehydration. After being treated in PBS (-) containing 0.3% (w/v) hydrogen peroxide, the sample was added to 2% BSA/PBS (-) solution containing 3% normal rabbit serum (DAKO) and then treated with an Avidin-Biotin blocking kit (Vector Laboratories). Subsequently, a slide carrying the sample thereon was treated overnight at 4°C with 10 µg/ml anti-Pim-3 IgY or IgY obtained before immunization and subsequently at room temperature for 30 minutes with 2.5 µg/ml biotin-conjugated rabbit anti-chicken IgY antibody (Promega). The resulting immunocomplex was visualized using a Vectastain Elite ABC kit (Vector Laboratories) and a Vectastain DAB substrate kit (Vector Laboratories) according to the instruction. Thereafter, the slide was costained with hematoxylin (DAKO) and observed under a microscope. Alternatively, normal liver tissue was subjected to the same procedures to create a comparison sample as a control.

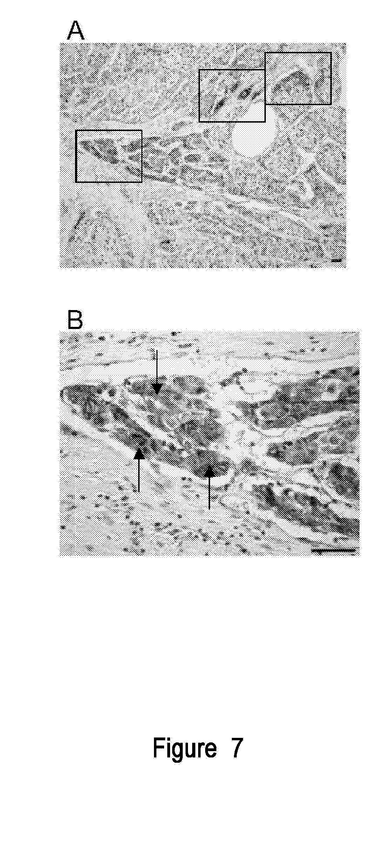

As a result, as shown in Figure 7, the hPim-3 polypeptide was not detected in the liver cancer tissue treated with the IgY before immunization (not shown), whereas immunoreactive activity was detected in many liver cancer cells from the liver cancer tissue treated with the anti-Pim-3 antibody, as with the regenerated bile duct epithelium (not shown). On the other hand, the hPim-3 polypeptide was not detected in the normal cell (not shown). These results indicate that Pim-3 is not expressed in normal liver cells and, in contrast, is specifically expressed in liver cancer cells.

In Figure 7, the plate B is an enlarged view of one of the boxed regions in the plate A and shows specifically stained liver cancer cells. The plates A and B are magnified by 40 times and 400 times, respectively.

### Example 9

### Preparation of siRNA

Based on the findings that hPim-3 is expressed specifically to liver cancer, siRNA (short interfering RNA) was first prepared by procedures described below. The synthesis of siRNA was performed using Silencer™ siRNA Construction Kit (Ambion) according to the instruction. At first, siRNA Target Finder (Ambion) and siRNA Design Tool (Ambion) were used to design an siRNA duplex molecule so that a target in mRNA was an AA(N19)UU sequence located in the mRNA open reading frame (ORF) coding for Pim-3. Namely, the selected target sequence for siRNA was an RNA portion having the nucleotide sequence of SEQ ID NO: 9. The RNA portion was subjected together with other human genome sequences to BLAST search to confirm the specificity of the target. The nucleotide sequences of the targeted mRNA and the created siRNA are as follows:

Nucleotide sequence (corresponding to cDNA) of targeted mRNA:
   5'-GCACGTGGTGAAGGAGCGCGG-3' (SEQ ID NO: 9)
Nucleotide sequence of siRNA:
   5'-CCGCGCUCCUUCACCACGUGC-3' (SEQ ID NO: 10)

Subsequently, a random RNA fragment (SEQ ID NO: 11) designed by B-Bridge International was adopted as a negative control.

Nucleotide sequence of random RNA fragment:
   5'-GCGCGCUUUGUAGGAUUCG-3' (SEQ ID NO: 11)

### Example 10

### Test of effect of hPim-3 siRNA

The siRNA and random RNA fragment of Example 9 were used to test the effect of siRNA.

At first, HuH7 cells (7x10⁵) were seeded into a cell culture dish of 6 cm in diameter and cultured for 2 days. The cells were mixed with 125 pmol each of the siRNA duplex molecule and the random RNA fragment, 12.5 µl of Lipofectamine 2000 (Invitrogen), and 2.5 ml of Opti-MEM (Invitrogen), and then left undisturbed at room temperature for 20 minutes. The mixture solution was directly added to the cells before saturation, which had been washed in advance with serum-free DMEM. On the following day, the resulting mixture was supplemented with 2.5 ml of DMEM containing 20% FBS and adjusted so that the final concentration of FBS was brought to 10%. After being left undisturbed for a given time, the cells were collected for subsequent analysis.

Two days after the transfection as described above, the cells were treated with trypsin and seeded at 5x10³ cells/well into a 96-well plate. The survival activity of the cells was measured daily using a WST-1 reagent (an MTT analog; Boehringer Mannheim Biochemicals). Cell growth analysis was performed by calculating the rate, with the 0-day value as a reference.

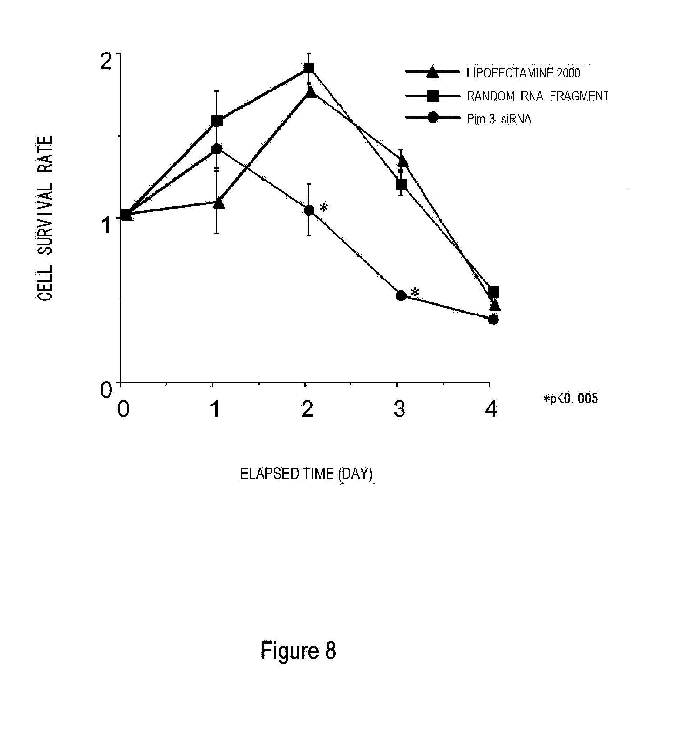

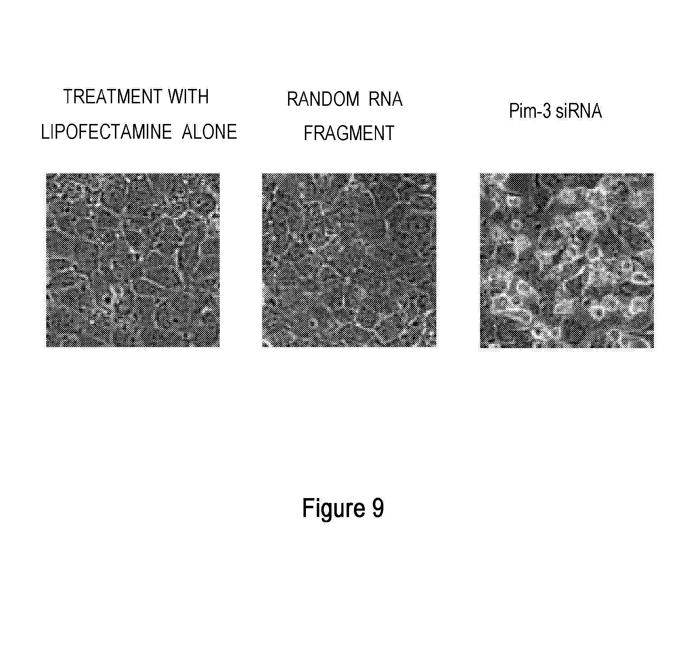

As a consequence of the analysis, as shown in the graph in Figure 8, the survival activity of the cell transfected with the Pim-3 siRNA was obviously reduced, as compared with the control transfected with the random RNA fragment. On the 4th day and subsequent days of the transfection, as shown in Figure 9, the cell did not exfoliate in the group treated with the random RNA fragment, whereas the cell started to exfoliate in the group treated with the Pim-3 siRNA. Thus, these results indicate that the survival activity of the human liver cancer cell HuH7 is reduced by treatment with Pim-3 siRNA.

### Example 11

### Cell cycle analysis of liver cancer cells into which hPim-3 siRNA was introduced

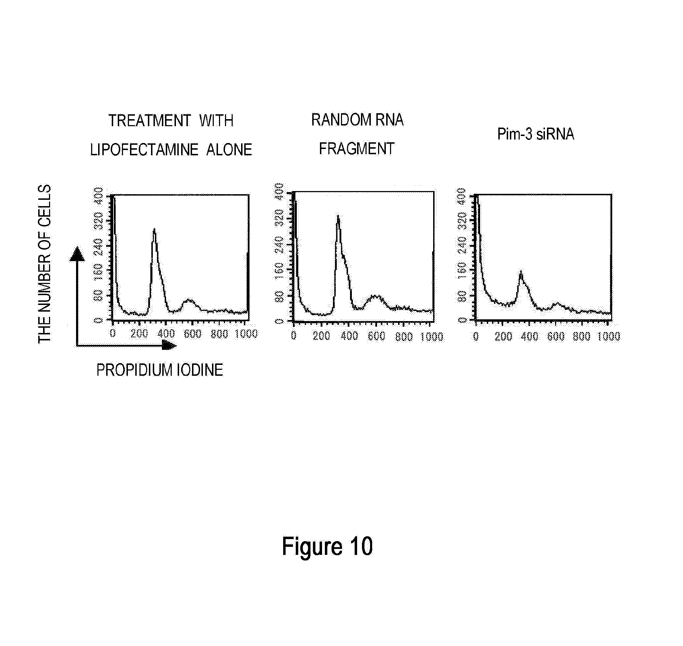

Liver cancer cells HuH7 into which hPim-3 siRNA was introduced in the same way as in Example 10 were used to perform cell cycle analysis by flow cytometry. Namely, the cells were collected 4 days after the transfection and fixed on ice using ethanol. Following the addition of 50 µg/ml propidium iodine and 1 µg/ml RNase A, the resulting cells were treated at room temperature for 30 minutes and supplemented with EDTA at a final concentration of 14 µM to terminate the reaction. After being filtered, the cells were analyzed using FACSCaliber (Becton Dickinson, Bedford, Mass.), and the distribution of their respective cell cycles was investigated using Cell Quest analysis software (Becton Dickinson). As a result, as shown in Figure 10, a reduction in G1 or G2/M phased cells and increased sub-Gl phased cells were observed in the liver cancer cells into which the Pim-3 siRNA was introduced, as compared with the cells into which the random mRNA fragment was introduced or the control cells. A reduction in the survival activity of the cancer cell HuH7 shown in Example 10 as well as changes in the cell cycle indicates that hPim-3 siRNA suppressed the expression of hPim-3 and induced the cell death of the cell HuH7.

The SEQ ID NOs in the sequence listing of the present specification represent the following sequences:

SEQ ID NO: 1 represents an amino acid sequence contained in the polypeptide specific to liver cancer of the present invention;
SEQ ID NO: 2 represents a nucleotide sequence coding for the polypeptide specific to liver cancer of the present invention and its regulatory region, and so on;
SEQ ID NO: 3 represents the nucleotide sequence of the Pim-3 sense primer used in Example 3;
SEQ ID NO: 4 represents the nucleotide sequence of the Pim-3 antisense primer used in Example 3;
SEQ ID NO: 5 represents the nucleotide sequence of the GAPDH sense primer used in Example 3;
SEQ ID NO: 6 represents the nucleotide sequence of the GAPDH antisense primer used in Example 3;
SEQ ID NO: 7 represents the nucleotide sequence of the probe used in the primary screening of hPim-3 in Example 4;
SEQ ID NO: 8 represents the amino acid sequence of the epitope used for producing the antibody against the hPim-3 polypeptide used in Example 7;
SEQ ID NO: 9 represents the nucleotide sequence (corresponding to cDNA) of the targeted mRNA in Example 9;
SEQ ID NO: 10 represents the nucleotide sequence of the siRNA of the present invention created in Example 9; and
SEQ ID NO: 11 represents the nucleotide sequence of the random RNA fragment used in Example 9.

(References)
1. Nakamoto, Y., L. G. Guidotti, C. V. Kuhlen, P. Fowler, and F. V. Chisari. 1998. Immune pathogenesis of hepatocellular carcinoma. J. Exp. Med. 188: 341-350.
2. Chisari, F. V., P. Fillipi, A. McLachlan, D. R. Milich, M. Riggs, S. Lee, R. D. Palmiter, C. A. Pinkert, and R. L. Brinster. 1986. Experssion of hepatitis B virus large envelope polypeptide inhibits hepatitis B surface antigen secretion in transgenic mice. J. Virol. 60: 880-887.
3. Wirth, S., L. G. Guidotti, K. Ando, H. J. Schlicht, and F. V. Chisari. 1995. Breaking tolerance leads to autoantibody production but not autoimmune liver disease in hepatitis B virus envelope transgenic mice. J. Immunol. 154: 2504-2515.
4. Moriyama, T., S. Guilhot, K. Klopchin, B. Moss, C. A. Pinkert, R. D. Palmiter, R. L. Brinster, O. Kanagawa, and F. V. Chisari. 1990. Immunobiology and pathogenesis of hepatocellular injury in hepatitis B virus transgenic mice. Science 248: 361-364.
5. Ito, T., and Y. Sakaki. 1999. Fluorescent differential display: a fast and reliable method for message display polymerase chain reaction. Methods enzymol. 303: 298-309.
6. Kitamura, K., Y. Nakamoto, M. Akiyama, C. Fujii, T. Kondo, K. Kobayashi, S. Kaneko, and N. Mukaida. 2002. Pathogenic roles of tumor necrosis factor receptor p55-mediated signals in dimethylnitrosamine-induced murine liver fibrosis. Lab. Invest. 82: 571-583.

## Claims

1. A polypeptide having at least 80% homology to the amino acid sequence of SEQ ID NO: 1; having an amino acid sequence, wherein at least an amino acid residue at position 39, 84,296, or 300 from the N-terminus is Ala, an amino acid residue at position 85 is Thr, an amino acid residue at position 163 or 333 is Ser, an amino acid residue at position 195 or 257 is Leu, an amino acid residue at position 271 is Arg, an amino acid residue at position 297 is Asp, an amino acid residue at position 313 is Pro, or an amino acid residue at position 316 is Val, in correspondence with the amino acid sequence of SEQ ID NO: 1; and having immunogenicity inducing the production of an antibody against a polypeptide comprising the amino acid sequence of SEQ ID NO: 1.

2. The polypeptide according to claim 1, wherein the homology is at least 90%.

3. The polypeptide according to claim 1, wherein the homology is at least 95%.

4. A polypeptide comprising the amino acid sequence of SEQ ID NO: 1.

5. A polypeptide fragment having a partial sequence of the amino acid sequence of SEQ ID NO: 1; or a partial sequence of an amino acid sequence having at least 80% homology to the amino acid sequence of SEQ ID NO: 1, wherein at least an amino acid residue at position 39, 84, 296, or 300 from the N-terminus is Ala, an amino acid residue at position 85 is Thr, an amino acid residue at position 163 or 333 is Ser, an amino acid residue at position 195 or 257 is Leu, an amino acid residue at position 271 is Arg, an amino acid residue at position 297 is Asp, an amino acid residue at position 313 is Pro, or an amino acid residue at position 316 is Val, in correspondence with the amino acid sequence of SEQ ID NO: 1; and having immunogenicity inducing the production of an antibody against a polypeptide according to claim 4.

6. A composition for producing an antibody specific to a polypeptide according to claim 4, comprising at least one selected from the group consisting of a polypeptide according to claim 1, a polypeptide according to claim 4, and a polypeptide fragment according to claim 5.

7. A method for the production of an antibody against a polypeptide according to claim 4, comprising administering a composition according to claim 6 to a mammal.

8. An antibody specifically binding to a polypeptide according to claim 4.

9. The antibody according to claim 8, wherein the antibody is a polyclonal antibody or a monoclonal antibody.

10. A diagnostic kit for liver cancer or a precancerous condition of the liver, comprising an antibody according to claim 8.

11. A polynucleotide coding for a polypeptide having at least 80% homology to the amino acid sequence of SEQ ID NO: 1; having an amino acid sequence, wherein at least an amino acid residue at position 39, 84, 296, or 300 from the N-terminus is Ala, an amino acid residue at position 85 is Thr, an amino acid residue at position 163 or 333 is Ser, an amino acid residue at position 195 or 257 is Leu, an amino acid residue at position 271 is Arg, an amino acid residue at position 297 is Asp, an amino acid residue at position 313 is Pro, or an amino acid residue at position 316 is Val, in correspondence with the amino acid sequence of SEQ ID NO: 1; and having immunogenicity inducing the production of an antibody against a polypeptide according to claim 4.

12. The polynucleotide according to claim 11, wherein the homology is at least 90%.

13. The polynucleotide according to claim 11, wherein the homology is at least 95%.

14. A polynucleotide coding for a polypeptide comprising the amino acid sequence of SEQ ID NO: 1.

15. A polynucleotide coding for a polypeptide fragment having a partial sequence of the amino acid sequence of SEQ ID NO: 1; or a partial sequence of an amino acid sequence having at least 80% homology to the amino acid sequence of SEQ ID No: 1, wherein at least an amino acid residue at position 39, 84, 296, or 300 from the N-terminus is Ala, an amino acid residue at position 85 is Thr, an amino acid residue at position 163 or 333 is Ser, an amino acid residue at position 195 or 257 is Leu, an amino acid residue at position 271 is Arg, an amino acid residue at position 297 is Asp, an amino acid residue at position 313 is Pro, or an amino acid residue at position 316 is Val, in correspondence with the amino acid sequence of SEQ ID NO: 1; and having immunogenicity inducing the production of an antibody against a polypeptide according to claim 4.

16. A polynucleotide comprising a nucleotide sequence from positions 436 to 1413 of SEQ ID NO: 2.

17. A vector comprising a polynucleotide according to any one of claims 11, 14, 15, and 16.

18. A host cell transformed with a vector according to claim 17.

19. A method for the production of a polypeptide according to claim 1 or claim 4 or a polypeptide fragment according to claim 5, comprising culturing a host cell according to claim 18 under a condition capable of producing the polypeptide or the polypeptide fragment and then collecting the polypeptide or the polypeptide fragment.

20. A PCR primer comprising at least 15 nucleotides corresponding to a polynucleotide coding for a polypeptide comprising the amino acid sequence of SEQ ID NO: 1.

21. A PCR primer comprising at least 15 nucleotides corresponding to a polynucleotide complementary to a polynucleotide coding for a polypeptide comprising the amino acid sequence of SEQ ID NO: 1.

22. A method for detecting a polynucleotide coding for a polypeptide comprising the amino acid sequence of SEQ ID NO: 1, comprising using PCR primers according to claim 20 and claim 21 to perform PCR.

23. A kit that detects a polynucleotide coding for a polynucleotide comprising the amino acid sequence of SEQ ID NO: 1, comprising PCR primers according to claim 20 and claim 21.

24. An RNA molecule comprising 15 to 25 nucleotide pairs, comprising a nucleotide sequence corresponding to a partial sequence of a nucleotide sequence from positions 436 to 1413 of SEQ ID NO: 2, or a mutant nucleotide sequence of the nucleotide sequence with the addition, deletion, or substitution of at least one base, and suppressing the expression of a protein specific to human liver cancer.

25. The RNA molecule according to claim 24, comprising 18 to 24 nucleotide pairs.

26. The RNA molecule according to claim 24, comprising 21 to 23 nucleotide pairs.

27. The RNA molecule according to claim 24, wherein the specific protein is a protein having the amino acid sequence of SEQ ID NO: 1 or a mutant protein thereof.

28. The RNA molecule according to claim 24, wherein the mutant protein is a protein having an amino acid sequence having 80% homology to the amino acid sequence of SEQ ID NO: 1.

29. The RNA molecule according to claim 24, wherein the mutant protein is a protein having an amino acid sequence having 90% homology to the amino acid sequence of SEQ ID NO: 1.

30. The RNA molecule according to claim 24, wherein the mutant protein is a protein having an amino acid sequence having 95% homology to the amino acid sequence of SEQ ID NO: 1.

31. An RNA molecule comprising the nucleotide sequence of SEQ ID NO: 10.

32. An RNA molecule comprising a mutant nucleotide sequence of the nucleotide sequence of SEQ ID NO: 10 with the addition, deletion, or substitution of at least one base, and suppressing the expression of a protein specific to human liver cancer.

33. The RNA molecule according to claim 24, wherein the RNA molecule has a hydroxyl group at the 3' end.

34. A pharmaceutical composition that suppresses the expression of a protein specific to human liver cancer, comprising an RNA molecule according to any one of claim 24 to claim 32.

35. A method for the production of a knockout cell, comprising introducing an RNA molecule according to any one of claim 24 to claim 32 to a cell expressing a protein specific to human liver cancer or a mutant protein thereof, and maintaining the cell under a condition bringing about RNA interference by the RNA molecule, to degrade mRNA transcribed from a gene coding for the specific protein or the mutant protein thereof.

36. A knockout cell produced by a method according to claim 34.

37. A kit for the functional analysis of a protein specific to human liver cancer, comprising an RNA molecule according to any one of claim 24 to claim 32.
